# EUROPÄISCHE PATENTSCHRIFT

(11) **EP 2 332 498 B1**
(45) Veröffentlichungstag und Bekanntmachung des Hinweises auf die Patenterteilung: **25.02.2015**
(21) Anmeldenummer: 10015424.4
(22) Anmeldetag: 08.12.2010
(51) Int. Cl.: A61F 2/78, A61F 2/50

(54) **Schutzeinrichtung insbesondere zur Verwendung für äussere Prothesen**
Protection device, in particular for use for external prosthetics
Dispositif de protection, notamment pour l'utilisation pour des prothèses extérieures

(30) Priorität: 08.12.2009 DE 102009057141
(43) Veröffentlichungstag der Anmeldung: 15.06.2011
(73) Patentinhaber: Sauer, Carsten, 51789 Lindlar (DE)
(72) Erfinder: Sauer, Carsten, 51789 Lindlar (DE)
(74) Vertreter: Rebbereh, Cornelia

(56) Entgegenhaltungen:
- WO-A1-00/51531
- DE-A1- 10 040 955
- DE-U- 1 789 684
- DE-U1- 20 120 916
- US-A- 5 133 775
- US-A- 5 593 453
- US-A- 5 769 809

## Beschreibung

Die Erfindung betrifft eine Schutzeinrichtung zur Verwendung für äußere Prothesen, wobei die Schutzeinrichtung ein maßlich anatomisch angepasster Schutzüberzug ist.

Äußere Prothesen in Form von Arm- und Beinprothesen sind bekannt. Diese bestehen insbesondere aus Metallen und/oder Carbon bzw. sonstigen Kunststoffen und Fasern. Diese Materialien eignen sich jedoch nicht besonders gut, um sie beim Duschen oder beim Wassersport zu tragen, da Wasser und insbesondere Salzwasser die hochwertigen und teuren Materialien der Prothese angreifen und beschädigen kann. Daher ist es bekannt, sog. Badeprothesen zu verwenden. Diese bestehen aus Kunststoffen, weisen jedoch weder im Tragekomfort noch in der Handhabung die Vorteile hochwertiger Prothesen aus Metall bzw. Carbon auf. Die eine Prothese tragende Person kann mit der Badeprothese duschen oder sich zu einem Badegewässer bewegen. Auch für kurze Wassergänge zur Abkühlung kann sie getragen werden. Es besteht jedoch das Problem, dass sie üblicherweise beim Schwimmen ausgezogen und am Ufer bzw. Einstiegsplatz in das Gewässer gelassen wird. Ferner besteht ein Problem darin, dass bei felsigen Ufern und unbewachten Stränden dies zumeist nicht möglich ist. Auch ist in dem Moment des Ablegens der Prothese das Handicap der betroffenen Person für Dritte ersichtlich und führt entsprechend zu für die betroffene Person belastendem Aufsehen. Aufgrund der üblicherweise verhältnismäßig einfachen Ausführung derartiger Badeprothesen wird die die Prothese tragende Person ferner daran gehindert, Gewässer aufzusuchen, die lediglich über Felsen, Steine und Pfade erreicht werden können, da auf diesen mit Badeprothesen in der Regel kein sicherer Halt möglich ist.

Bekannt sind ferner Schläuche aus dünnem Material, die durch einen Gummizug oder ähnliches einseitig zusammengezogen werden können. Um diese an die anatomische Form der die Prothese oder einen Gips tragenden Person anpassen zu können, wird der Schlauch im Inneren unter Unterdruck gesetzt. Hierfür sind ein Ventil sowie ein Pumpball in der Schlauchhaut oder eine andere Art einer Einrichtung zum Erzeugen eines Unterdrucks vorgesehen. Eine solche Lösung für die Abdichtung eines Gipsverbades ist beispielsweise aus der AT 61218E oder von der Firma Dry Corp., LLC unter der Bezeichnung Dry Pro™ bekannt. Alternativ ist nach Art eines Schrumpfschlauches ein Anlegen an den Körper der die Prothese bzw. den Gips tragenden Person durch Hitze bekannt. In jedem der Fälle wird ein ästhetisch wenig ansprechendes Ergebnis erzielt. Ferner besteht bei dem Vorsehen eines Ventils mit Pumpball bei der späteren Verwendung das Problem, dass das Ventil beschädigt oder geöffnet werden kann, so dass keine Dichtwirkung mehr gegeben ist. Ferner wird auch ein Schwimmen in Gewässern aufgrund des Übermaßes an Material behindert. Ein weiteres Problem stellt die geringe Langlebigkeit dieser Schläuche bzw. Schrumpfschläuche dar, da diese sehr schnell beschädigt und somit undicht werden können. Bei einem Ventilausfall bzw. Druckverlust bzw. bei Beschädigung des Schlauchs dringt in kurzer Zeit eine große Wassermenge in dessen Inneres ein. Die Schrumpfschlauchvariante lässt sich ferner nur einmalig verwenden und muss danach mühevoll wieder entfernt werden. Diese Variante stellt also ein Wegwerfprodukt dar, das nach dem Gebrauch nur mühsam wieder entfernt werden kann.

Aus der DE 41 25 635 A1 sind ferner ein Überzug für eine Oberschenkel- oder Unterschenkel-Prothese und ein Verfahren zu seiner Herstellung bekannt, wobei der Überzug aus einem elastischen Material mit einer geschlossenen dichten Oberfläche besteht, so dass der Überzug wasserundurchlässig ist und eine Wasseraufnahme vermieden wird. Der Überzug ist schlauchartig mit einer den zu ersetzenden Gliedmaßen formmäßig entsprechenden Gestalt ausgebildet. Er kann mit seinen beiden voneinander entfernten Öffnungsabschnitten an den entsprechenden Teilen der Prothese befestigt werden. Es wird also nur die Prothese wasserundurchlässig versiegelt, also im Grund eine Art Badeprothese aus einer normalen Prothese geschaffen. Der Übergang des oberen Prothesenrandes zur Haut der die Prothese tragenden Person ist jedoch nicht wasserdicht oder in irgendeiner Weise vor dem Eindringen von Wasser in das Innere des Überzugs oder in die Prothese geschützt ausgebildet, so dass Wasser dort eintreten kann. Ohne die außenseitige Versiegelung ist das Material des Überzugs ferner nicht wasserdicht, sondern nimmt sogar Wasser auf, da hier ein normales Textil über die Prothese gezogen und wasserdicht versiegelt wird.

Die US 5,593,453 A offenbart einen Prothesenüberzug aus einem wasserdichten Latex-Material, das sich eng an die zu überdeckende Beinform anlegt. Auf der Innenseite weist der Überzug eine rutschfeste Oberfläche auf. Ferner weist der Überzug bei Ausbilden als Überzug für ein Bein eine rutschfeste Sohle auf. Die rutschfeste Oberfläche ist durch Vorsehen einer Anzahl von nach innen weisenden Rippen auf der Innenseite des Beinteils des Überzugs gebildet, wobei die Anzahl von Rippen voneinander beabstandet sind und jede Rippe sich konzentrisch zu dem Beinteil erstreckt. Die Rippen erstrecken sich von über einem Fußknöchelbereich bis zu einem oberen Abschnitt, um den Überzug von der Prothese zu beabstanden und hierdurch einen Reibeingriff beim Überstreifen des Überzugs über die Prothese durch ein Gleiten über diese zu vermindern. Außenseitig weist der Überzug Schlaufen zum Erleichtern des An- und Ausziehens auf, die allerdings den ästhetischen Eindruck negativ beeinflussen und nicht die Anmutung menschlicher Haut vermitteln.

Der vorliegenden Erfindung liegt nun die Aufgabe zugrunde, die bezüglich der bekannten Schutzeinrichtungen genannten Problematiken zu überwinden und eine Einrichtung als Ersatz für eine Badeprothese sowie evakuierbare oder Schrumpfschläuche sowie die genannten Schutzüberzüge zu schaffen, die ein sicheres Duschen und Baden sowie Wassersport und eine aktive und unauffällige Teilnahme am Strandleben ohne Einschränkungen zulässt, wobei ein Eindringen von Wasser und Fremdkörpern, wie Sand, Schmutz und Staub, in das Innere der Schutzeinrichtung sicher vermieden werden kann.

Die Aufgabe wird durch eine Schutzeinrichtung gemäß Anspruch 1 gelöst. Weiterbildungen der Erfindung sind in den abhängigen Ansprüchen definiert. Dadurch wird eine Schutzeinrichtung zur Verwendung für äußere Prothesen geschaffen, die als Schutzüberzug über eine herkömmliche Prothese getragen wird. Hierdurch stellt sie einen Schutz für diese dar, da das Eindringen von Wasser ebenso wie Fremdkörpern und somit eine Beschädigung der Prothese durch das eindringende Wasser bzw. eindringende Fremdkörper, wie insbesondere auch Sand, erfolgreich vermieden werden kann. Unter dem Begriff der Wasserundurchlässigkeit werden sowohl eine Wasserdichtigkeit als auch ein Spritzwasserschutz und eine Wasserfestigkeit verstanden. Das Merkmal eines anwendungsspezifisch ausreichend ein Eindringen von Wasser in das Innere des Schutzüberzugs Verhinderns bedeutet hier, dass je nach Anwendung der Schutz durch das Material der Schutzeinrichtung sowie diese selbst so ausgelegt ist, dass ein Eindringen von Wasser oder Feuchtigkeit in das Innere der Schutzeinrichtung verhindert werden kann. Das Material der Schutzeinrichtung ist dabei wasserdicht, der Aufbau der Schutzeinrichtung sowie deren sonstige Verarbeitung z.B. im Bereich von Materialstoßstellen verhindert anwendungsspezifisch bzw. im Hinblick auf die jeweilige Anwendung das Eindringen von Wasser in das Innere der Schutzeinrichtung. Hierbei kann beispielsweise Spritzwasser, jedoch auch Wasser bei einem Dusch- oder Badevorgang von einem Eindringen in das Innere der Schutzeinrichtung sicher abgehalten werden. Hierzu dient die manschettenartige zumindest teilelastische Einrichtung, die einen festen Halt der Schutzeinrichtung auf der Haut der die Prothese tragenden Person dadurch ermöglicht, dass sie sich an die Formgebung des der Prothese benachbarten Körperteils, auf dem der Schutzüberzug sich festhält, optimal anpasst ist. Durch diese Formanpassung in Verbindung mit der zumindest Teilelastizität hält sie auf der Haut der die Schutzeinrichtung tragenden Person optimal fest. Der Schutzüberzug gemäß der US 5,593,453A weist lediglich einen konisch zulaufenden oberen Endabschnitt auf, jedoch ist dieser einteilig mit dem restlichen Überzug. Es ist also keine manschettenartige Einrichtung angesetzt, die aus einem einen besonderen Halt auf dem Extremitätenstumpf gebenden, zumindest teilelastischen Material besteht, wie dies bei der vorliegenden Erfindung der Fall ist. Bei Verwendung der Schutzeinrichtung gemäß der vorliegenden Erfindung ist also die Prothese durch das für den Schutzüberzug verwendete Material optimal gegen Beschädigungen geschützt, und die Prothese ist ferner durch Vorsehen der manschettenartigen, zumindest teilelastischen Einrichtung, die an den Rest des Schutzüberzugs angefügt ist, optimal gegen eine Beschädigung durch in das Innere der Schutzeinrichtung eindringendes Wasser, Feuchtigkeit oder Fremdkörper etc. geschützt, so dass die manschettenartige Einrichtung und der restliche Teil bzw. der Hauptkörper des Schutzüberzugs jeweils aus einem optimalen Material bestehen können, die aneinander gesetzt werden. Die Prothese ist somit in jeder Hinsicht optimal geschützt.

Durch die Verwendung eines zumindest teilelastischen Materials, also eines Materials, das zumindest teilweise Elastizität aufweist und das maßlich anatomisch angepasst ist, liegt die Schutzeinrichtung zumindest an den relevanten Stellen eng an dem Extremitätenstumpf, an den die Prothese angegliedert ist, und ggf. auch an der Prothese an, so dass die Schutzeinrichtung an der gewünschten Stelle verbleibt und bereits hierdurch ein Eindringen von zumindest Schmutz, wie Staub, jedoch auch Sand vermieden werden kann. Unter der maßlich anatomischen Anpassung wird das maßliche Abstimmen auf die Anatomie eines Menschen verstanden, nicht unbedingt eines bestimmten Menschen, sondern allgemein an die anatomischen Formgebungen und Abmessungen von Menschen. Es sind eine Serienfertigung, jedoch selbstverständlich auch eine individuelle Anpassung an eine bestimmte Person hier möglich. Bei einer Serienfertigung kann die Schutzeinrichtung in verschiedenen Größen, angepasst an die Anatomie unterschiedlich großer und dicker Personen, angeboten werden, vom Prinzip her wie bei verschiedenen Konfektionsgrößen bei Bekleidungsstücken. Es kann hierdurch auch optisch eine Anpassung an den Körper der die Schutzeinrichtung tragenden Person geschaffen werden, somit ein ästhetischer Vorteil gegenüber den Schläuchen des Standes der Technik, die keine maßliche anatomische Anpassung aufweisen, sondern vielmehr im reichlichen Übermaß ausgebildet sind und entsprechend nach dem Evakuieren bzw. Schrumpfen lappenartig überstehen über die anatomische Form der Person, insbesondere im Bereich eines Armes oder Beines der Person.

Durch die Verwendung eines widerstandsfähigen und beschädigungsresistenten Materials kann verhindert werden, dass bei der Verwendung der Schutzeinrichtung Risse auftreten und somit dann doch Nässe und Fremdkörper in das Innere der Schutzeinrichtung eindringen können. Unter einem widerstandsfähigen und beschädigungsresistenten Material wird ein Material verstanden, das verhältnismäßig reißfest ist und dick genug, um beispielsweise bei Vorbeistreifen an Steinen oder anderen harten Gegenständen nicht einzureißen. Widerstandsfähig bedeutet in diesem Zusammenhang, dass das Material in der Lage ist, Belastungen zu ertragen, ohne oder zumindest im Wesentlichen ohne Schaden zu nehmen, insbesondere ohne einzureißen. Beschädigungsresistent bedeutet in diesem Zusammenhang insbesondere, dass das Material lediglich durch gezieltes Zerschneiden getrennt werden kann, ansonsten jedoch stabil genug ist, um die die Schutzeinrichtung tragende Person sicher gegen ein Eindringen von Feuchtigkeit und/oder Fremdkörpern zu schützen. Als besonders geeignet erweisen sich hier wasserdichte, nicht wasseraufnehmende Materialien, wie Chloropren-Kautschuk, Polychloropren-oder Chlorbutadien-Kautschuk, also geschäumte Gummimaterialien. Latexmaterial ist in diesem Sinne kein beschädigungsresistentes und widerstandsfähiges Material. Die im Stand der Technik genannten Materialien, wie insbesondere Latex, weisen die gewünschte Stabilität und Widerstandsfähigkeit nicht auf, insbesondere ist ein ungezwungenes Bewegen und Herumtollen am Strand mit diesen Schutzeinrichtungen daher nicht möglich. Ferner können über die Schutzeinrichtungen des Standes der Technik keine Badeschuhe gezogen werden. Dies hat einen entscheidenden Nachteil, da bei einer normalen Prothese das Fußgelenk immer in einem bestimmten Winkel eingestellt ist, abhängig von der gewünschten Absatzhöhe. Ohne Schuh kommt das Bein bei Unachtsamkeit automatisch in eine überstreckte Position. Damit kann man zwar problemlos schwimmen, jedoch gefahrlos laufen oder gehen nur, wenn man einen Schuh trägt, was bei der Schutzeinrichtung gemäß der vorliegenden Erfindung möglich ist, nicht jedoch bei den Schutzeinrichtungen des Standes der Technik, wie sie vorstehend genannt sind. Ein problemloses Gehen oder Laufen am Strand, insbesondere ein Herumtollen, und auch Gartenarbeit kann eine Person mit den Schutzeinrichtungen des Standes der Technik somit nicht ausführen.

Besonders vorteilhaft ist die manschettenartige Einrichtung an das für die restliche Erstreckung der Schutzeinrichtung verwendete Material angesetzt, also als Manschette endseitig angesetzt. Hierdurch ist es möglich, die zum Inneren der Schutzeinrichtung gerichtete Oberfläche der manschettenartigen Einrichtung haftend auszubilden bzw. mit einer Adhäsivwirkung zu versehen. Ein Eindringen von Nässe bzw. Feuchtigkeit wird durch die manschettenartige Einrichtung sicher vermieden. Sie umfasst die Öffnung, durch die in die Schutzeinrichtung hineingeschlüpft wird, also das Ende der Schutzeinrichtung, das nachfolgend an dem Extremitätenstumpf anliegt. Aufgrund der anatomisch angepasst bemessenen Einrichtung liegt diese besonders eng und passend an dem Extremitätenstumpf an, so dass bereits aus diesem Grunde ein Eindringen von Nässe und Fremdkörpern, insbesondere auch Staub, in die Schutzeinrichtung vermieden werden kann.

Die manschettenartige Einrichtung kann ein lang anhaltend elastisches Material mit einer Adhäsivwirkung, insbesondere Latex und/oder Silikon, umfassen und/oder ein Material mit glatter, Hafteigenschaft aufweisender Oberfläche. Aufgrund der Materialelastizität legt sich, wie bereits erwähnt, die manschettenartige Einrichtung bereits sehr eng an der Oberfläche der zu der Prothese benachbarten Haut der die Prothese tragenden Person an, so dass hierdurch ein guter Schutz gegen eindringende Feuchtigkeit, aber auch gegen ein Eindringen von Staub gegeben ist. Die Schutzeinrichtung lässt sich im Unterschied zu einem Schrumpfschlauch mühelos nach dem Gebrauch wieder ablegen und somit mehrfach wiederverwenden.

Wie erwähnt, besteht die Schutzeinrichtung vorteilhaft zumindest teilweise aus einem Chloropren-Kautschuk, Polychloropren- oder Chlorbutadien-Kautschuk. Im end- bzw. randseitigen Bereich bzw. im Bereich der manschettenartigen Einrichtung der Schutzeinrichtung, der so ausgebildet ist, dass er die Haut der die Prothese tragenden Person überdecken kann, kann der Chloropren-Kautschuk bzw. das Polychloropren- oder der Chlorbutadien-Kautschuk auf der zur Auflage auf der Haut der Person vorgesehenen Seite mit einer glatten, Hafteigenschaft aufweisenden Oberfläche versehen, z.B. als sog. Glatthaut-Neopren^{®} ausgebildet sein. Bei Vorsehen eines solchen Rand- bzw. Kantenabschlusses bzw. bei Vorsehen der manschettenartigen Einrichtung und Ansetzen an das sonstige Material der Schutzeinrichtung kann ein Eindringen von Wasser in das Innere der Schutzeinrichtung bei Aufliegen dieses Randabschlusses bzw. der manschettenartigen Einrichtung auf der Haut der die Prothese tragenden Person unterbunden werden.

Zum Schaffen eines guten Haltes auf der Haut einer die Schutzeinrichtung tragenden Person kann die Einrichtung in Form eines Randabschlusses bzw. in Form der angesetzten Manschette mit einem Keil aus Silikon oder Latex versehen sein oder selbst aus Silikon- oder Latexmaterial bestehen. Da die Einrichtung vorteilhaft eine Adhäsivwirkung, also eine haftende oder Klebewirkung aufweist, ist durch diese adhäsive Wirkung bereits ein sehr guter und dichter Halt auf der Hautoberfläche der Person möglich.

Um eine besonders gute Wasserdichtigkeit und einen noch besseren Halt auf der Hautoberfläche der Person vorzusehen, kann ein Hautkleber auf den glatten, Hafteigenschaften aufweisenden Randbereich der Schutzeinrichtung, also die manschettenartige Einrichtung aufgetragen werden. Dieser kann flüssig, ggf. in Form eines doppelseitigen Klebebandes oder in einer anderen Form eines Klebemittels ausgebildet sein. Er wird auf der zur Auflage auf der Haut der Person vorgesehenen Seite bzw. der Innenseite der als Schutzüberzug ausgebildeten Schutzeinrichtung endseitig dort vorgesehen, wo eine Öffnung zum Eintritt in bzw. Austritt aus dem Schutzüberzug vorgesehen ist. Bei einem Schutzüberzug in der Form eines lang ausgebildeten Strumpfes zum Schutz einer Beinprothese wird die Einrichtung bzw. der Hautkleber vorteilhaft in dem Bereich, in dem der Schutzüberzug auf der Haut der die Prothese tragenden Person aufliegt, vorgesehen.

Alternativ zum Vorsehen eines Hautklebers, oder ggf. zusätzlich zu diesem, kann ein separates manschettenartiges Abdeckelement aus einem wasserdichten oder ein Eindringen von Wasser verhindernden Material, insbesondere aus Silikon und/oder Latex, vorgesehen sein. Dieses kann sowohl einen Randbereich der Schutzeinrichtung als auch die Haut der die Schutzeinrichtung tragenden Person überdecken und somit einen Schutz vor dem Eindringen von insbesondere Feuchtigkeit bzw. Nässe sowie Staub bieten. Ein Haftwirkung aufweisendes Material kann beispielsweise auf der Außenseite der endseitigen manschettenartigen Einrichtung der Schutzeinrichtung aufgedruckt sein, so dass bei Überdecken der manschettenartigen Einrichtung durch das manschettenartige Abdeckelement eine wasserdichte Verbindung möglich ist. Es ist somit sowohl durch ein Verkleben der Schutzeinrichtung auf der Haut der die Prothese tragenden Person als auch durch Vorsehen des manschettenartigen Abdeckelements möglich, ein Eindringen von Wasser und Fremdkörpern in die Schutzeinrichtung noch sicherer zu vermeiden, sogar bei verschiedensten Arten von Wassersport. Bei Vorsehen einer Latex- oder Silikonmanschette als randseitigem Abschluss der Schutzeinrichtung, also als an die Schutzeinrichtung angefügtem Randabschluss bzw. manschettenartige Einrichtung oder als separatem Abdeckelement kann eine wasserdichte Befestigung auf der Haut einer Person auch ohne einen Hautkleber erfolgen.

Das Aufrollen des endseitigen Randbereichs, also des Randabschnitts bzw. der manschettenartigen Einrichtung, beim Tragevorgang durch eine Person kann durch das Aufkleben auf der Haut der Person verhindert werden. Ferner ist es jedoch möglich, die Einrichtung mit zumindest einer eine Aufrollbewegung verhindernden Einrichtung zu versehen. Diese kann als zumindest eine taschenartige Aufrollverhinderungseinrichtung mit zumindest einer stabartigen Stützeinlage ausgebildet sein. Die taschenartige Aufrollverhinderungseinrichtung kann auf das Material der Schutzeinrichtung aufgefügt werden, z.B. in Form eines Materialflecks aufgebügelt, bei Ausbilden aus Gummimaterial oder bei Beschichten mit einem adhäsiven Material haftend aufgefügt oder in sonstiger Weise aufgebracht sein. Zum Aussteifen, um also eine Rollbewegung des Randabschnitts der Schutzeinrichtung zu vermeiden, wird zumindest eine Stützeinlage z.B. in Form eines Stäbchens in die taschenartige Aufrollverhinderungseinrichtung eingefügt. Durch das nachträgliche Einfügen ist es möglich, die Stützeinlage zum Reinigen der taschenartigen Aufrollverhinderungseinrichtung wieder zu entnehmen. Es kann jedoch alternativ auch zumindest eine Stützeinrichtung vorgesehen werden, die nicht entnehmbar an dem Material der Schutzeinrichtung befestigt ist/wird.

An dieser Stelle soll betont werden, dass das Vorsehen eines Hautklebers, eines separaten manschettenartigen Abdeckelements oder einer Aufrollverhinderungseinrichtung vorteilhafte Zusatzeinrichtungen sind, die Schutzeinrichtung jedoch auch ohne diese auskommt. Durch diese Zusatzeinrichtungen kann die Wasserdichtigkeit für bestimmte Einsatz- oder Anwendungsfälle weiter verbessert werden, bei denen größere ein Lösen der Schutzeinrichtung von der Hautoberfläche der diese tragenden Person bewirkende Kräfte auf die Schutzeinrichtung einwirken, wie z.B. beim Tauchen oder einer stark konischen Körperformgebung im Bereich des Extremitätenstumpfes der die Schutzeinrichtung tragenden Person.

Weiter ist die Schutzeinrichtung mehrlagig ausgebildet. Hierbei kann die Schutzeinrichtung zumindest eine geschäumte Lage aufweisen, beispielsweise eine Lage aus geschäumtem Chloropren-Kautschuk, Polychloropren- oder Chlorbutadien-Kautschuk. Um eine robuste und gut handhabbare Oberfläche vorzusehen, weist die Schutzeinrichtung weiter vorteilhaft zumindest einseitig eine Oberflächenbeschichtung auf, ist insbesondere kaschiert. Eine solche Oberflächenbeschichtung bzw. Kaschierung kann einseitig oder beidseitig vorgesehen sein, also auf der Außen- und/oder Innenseite der Schutzeinrichtung. Grundsätzlich ist es ebenfalls möglich, keine Oberflächenbeschichtung bzw. Kaschierung vorzusehen. Als Materialien für eine solche Oberflächenkaschierung eignen sich insbesondere Lycra und Nylon, die z.B. als Gewebefläche aufgebracht sind.

Der endseitige Randabschluss bzw. die manschettenartige Einrichtung der Schutzeinrichtung kann aus einem Chloropren-Kautschuk, Polychloropren- oder Chlorbutadien-Kautschuk in kaschierter oder unkaschierter Ausführung bestehen. Auf der zu der die Schutzeinrichtung tragenden Person gerichteten Seite ist diese vorteilhaft, wie bereits erwähnt, als sog. Glatthaut-Neopren^{®} ausgebildet, wobei sie dabei auf der einen Seite eine Kaschierung aufweist und auf der anderen Seite unkaschiert ist und eine glatte Oberfläche des Chloropren-Kautschuks, Polychloropren- oder Chlorbutadien-Kautschuks aufweist, um zu der Haut der die Schutzeinrichtung tragenden Person gerichtet die gewünschte Adhäsivwirkung aufzuweisen. Bei Vorsehen eines endseitigen Randabschlusses bzw. einer manschettenartigen Einrichtung aus Silikon und/oder Latex ist dieser/diese vorteilhaft als Manschette in Vollmaterial ausgebildet. Sofern technisch machbar, können diese Manschetten zusätzlich auf der von der die Schutzeinrichtung tragenden Person wegweisenden Seite kaschiert sein. Die Einrichtung zum Schutz vor Nässe bzw. Feuchtigkeit sowie vor dem Eindringen von Staub, Sand bzw. Schmutz kann somit entweder als Manschette aus insbesondere den vorstehend genannten Materialien vorgesehen und mit dem übrigen Material der Schutzeinrichtung verbunden bzw. an diesem befestigt werden oder ggf. direkt mit an das übrige Material der Schutzeinrichtung angeformt oder darauf aufgefügt sein.

Die Stoßstellen des Materials der Schutzeinrichtung können im Wesentlichen wasserdicht verschlossen, insbesondere vernäht, verschweißt oder verklebt sein/werden. Auch eine Mischung dieser Verbindungsarten ist ohne weiteres möglich. Das für die Herstellung der Schutzeinrichtung verwendete Material kann eine Materialstärke von beispielsweise 0,5 mm bis 5 mm aufweisen, insbesondere von 1 bis 3 mm. Hierdurch ist eine sehr gute Stabilität der Schutzeinrichtung möglich in Verbindung mit einer nicht zu großen Steifigkeit und hierbei dennoch möglichen Formstabilität. Gerade bei Verwendung eines dünnen Materials für die Schutzeinrichtung, also insbesondere eines dünnen Chloropren-Kautschuks, Polychloropren- oder Chlorbutadien-Kautschuks kann dieses Material auf Stoß verschweißt und mit entsprechenden Bändern die jeweilige Schweißstelle abgedeckt werden. Hierdurch ist es möglich, auch bei Verwendung dünneren Materials eine ausreichende Wasserdichtigkeit und Widerstandsfähigkeit gegen Beschädigungen vorzusehen. Zusätzlich zu der geschäumten Lage können noch andere Materiallagen vorgesehen werden, die aufeinander gefügt das Material der Schutzeinrichtung ergeben. Insbesondere können außenseitig auch besonders abriebfeste und beispielsweise gegen Seewasser besonders stabile Materialschichten vorgesehen werden.

Ferner kann im Bereich der Sohle eines Fußes zumindest ein Reibung erhöhendes Material oder Materialflecken vorgesehen werden, um insbesondere beim Überschreiten von Felsen oder anderem rutschigen Gelände einen guten Halt zu ermöglichen. Die Fußsohle wird also rutschfest ausgebildet, insbesondere beschichtet.

Durch die dichte Ausbildung der Schutzeinrichtung selbst und deren eng anliegende Anordnung an der Haut der die Prothese tragenden Person, auch bereits ohne Verkleben oder Verwendung eines separaten manschettenartigen Abdeckelements, kann nicht nur ein Eintritt von Wasser, sondern selbstverständlich auch von Sand und Staub in das Innere der Schutzeinrichtung wirksam verhindert werden, so dass auch eine Beschädigung der Prothese hierdurch erfolgreich vermieden werden kann. Wie bereits erwähnt, ist eine Verwendung weiterer Hilfsmittel zum Erzielen der Wasserdichtigkeit bzw. der Dichtheit gegen ein Eindringen von Staub und Sand daher nicht erforderlich.

Die Schutzeinrichtung ist als hand- oder fußintegrierender Arm- oder Beinling ausgebildet sein. Ebenfalls ist es möglich, die Schutzeinrichtung als schlauchförmigen, hand- oder fußlosen Arm- oder Beinling auszubilden. Ein besonders sicherer Schutz vor dem Eindringen von Wasser bzw. Feuchtigkeit kann bei dem Integrieren von Hand oder Fuß in die Schutzeinrichtung erzeugt werden, da dann lediglich eine Öffnung sicher abzudichten und zu verschließen ist. Bei Vorsehen einer schlauchförmigen, jedoch maßlich an die diese tragende Person angepassten Schutzeinrichtung, bei der Hand bzw. Fuß der die Prothese tragenden Person außerhalb der Schutzeinrichtung liegen, sind zwei Öffnungen an der Schutzeinrichtung vorgesehen, die am Handgelenk bzw. dem Knöchel bzw. im Bereich von Hand oder Fuß der die Prothese tragenden Person sowie im Bereich der Schulter bzw. des Oberschenkels oder der Hüfte der die Prothese tragenden Person angeordnet und insbesondere verklebt werden.

Die Schutzeinrichtung kann ferner als Teil des Körpers der die Schutzeinrichtung tragenden Person integrierender einbeiniger hosenartiger oder oberkörperintegrierender Überzug ausgebildet sein. Eine derartige Ausbildung eignet sich besonders bei Personen, bei denen lediglich sehr kurze Stümpfe im Bereich der Extremitäten verblieben sind, so dass ein sicheres Befestigen der Schutzeinrichtung dort kaum möglich ist. Zu diesem Zweck wird die Schutzeinrichtung dann als hosenartiger bzw. oberkörperintegrierender Überzug ausgebildet, so dass der Halt am übrigen Körper und nicht am Extremitätenstumpf erfolgt. Auch hier kann an den jeweiligen Öffnungen, an denen ein Eindringen von Nässe bzw. Feuchtigkeit oder auch Staub und Sand möglich ist, ein haftendes Material bei Vorsehen einer entsprechenden manschettenartigen Einrichtung und ggf. ein Hautkleber aufgebracht werden, um Wasserdichtigkeit oder zumindest ein Hindernis für das Eindringen von Nässe, Feuchtigkeit, Staub, Sand und Fremdkörpern zu schaffen.

Optisch kann die Schutzeinrichtung entweder an die normale Hautfarbe der diese tragenden Person angepasst oder anderweitig farbig ausgebildet sein. Dies ist im Unterschied zu den im Stand der Technik bekannten Materialien möglich und führt zu einem optisch sehr ansprechenden Ergebnis. Zum Einfärben des Materials können herkömmliche Verfahren, wie Siebdruck oder auch Lackierungen sowie Airbrush verwendet werden. Ebenso kann die Eigenfärbung einer Kaschierung auf der nach außen, also von der die Schutzeinrichtung tragenden Person weg gerichteten Seite genutzt werden, die in unterschiedlichen Farben vorgesehen werden kann. Grundsätzlich kann auch das für die Schutzeinrichtung verwendete Material selbst eingefärbt werden.

Die Schutzeinrichtungen können aus einem Chloropren-Kautschuk, Polychloropren- oder Chlorbutadien-Kautschuk als Materialzuschnitt geschnitten und nachfolgend dicht an ihren Stoßstellen gefügt werden, beispielsweise durch Vernähen, Verkleben oder Verschweißen. Das Aufbringen einer Kaschierung oder Beschichtung erfolgt bereits auf dem Grundmaterial oder ggf. vor dem Fügen entlang den Stoßstellen der Materialzuschnitte der Beinlinge bzw. Armlinge. Eine Beschichtung kann hier beidseitig oder auch lediglich einseitig vorgesehen sein oder werden, so dass bei den fertigen Beinlingen bzw. Armlingen entweder nur auf der Außenseite oder nur auf der Innenseite oder auf der Außen- und Innenseite eine Beschichtung durch Auffügen einer weiteren Materiallage bzw. Aufkaschieren einer solchen vorgesehen ist. Die Materialspezifikation bzw. -ausführung, also Farbe, konkretes Material, einseitige oder zweiseitige Kaschierung bzw. Beschichtung, einseitige Glatthautausführung, etc., kann bereits bei dem Grundmaterial berücksichtigt werden, also bei einer Meterware oder einem Materialzuschnitt. Eine Lage aus einem geschäumten Chloropren-Kautschuk, Polychloropren- oder Chlorbutadien-Kautschuk, beispielsweise von DuPont vertrieben unter der Marke Neopren^{®}, wird z.B. als mittlere Schicht eines mehrlagigen Aufbaus des Materials der Schutzeinrichtung vorgesehen. Sie kann jedoch auch als alleinige Schicht vorgesehen werden. Es sind somit die Varianten des Vorsehens lediglich einer teilelastischen und im Wesentlichen wasserundurchlässigen Materialschicht, einer solchen mit nur nach außen, also weg von der die Schutzeinrichtung tragenden Person gerichteten Kaschierung oder Beschichtung, einer solchen mit nur nach innen, also zu der die Schutzeinrichtung tragenden Person gerichteten Kaschierung oder Beschichtung und einer solchen mit einer äußeren und einer inneren Beschichtung bzw. Kaschierung möglich. Für die Randabschlüsse bzw. Manschetten kann, wie bereits erwähnt, ein Vollmaterial aus einem Hafteigenschaften aufweisenden Material, wie Latex oder Silikon vorgesehen werden. Ferner kann das Grundmaterial der Schutzeinrichtung in der Ausführung mit glatter Oberfläche auf der inneren, zu der die Schutzeinrichtung tragenden Person gerichteten Seite vorgesehen werden, wobei hier insbesondere auch noch eine außenseitige Kaschierung bzw. Beschichtung vorgesehen werden kann. Nicht nur das Grundmaterial, sondern auch die Randabschlüsse bzw. manschettenartigen Einrichtungen können vorproduziert und lediglich anwendungsspezifisch zugeschnitten und mit dem übrigen Material der Schutzeinrichtung verbunden werden.

Bei verletzten Gliedmaßen werden Wassersport sowie ein Strandbesuch vermieden, um die Wunde vor einem Eindringen von Feuchtigkeit und Schmutz zu schützen. Um es einer verletzten Person dennoch zu ermöglichen, aktiv am Strandleben sowie ggf. sogar am Wassersport teilzunehmen, kann eine Schutzeinrichtung zum Schutz von Gliedmaßen gegen einen Kontakt mit Feuchtigkeit und insbesondere Fremdkörpern, wie Schmutz, vorgesehen sein, die ein maßlich anatomisch angepasster Schutzüberzug aus einem zumindest teilelastischen, wasserundurchlässigen oder anwendungsspezifisch ausreichend ein Eindringen von Wasser in das Innere des Schutzüberzugs verhindernden, widerstandsfähigen und beschädigungsresistenten Material ist und zumindest an einem Ende mit einer vor Eintritt von Nässe oder Feuchtigkeit sowie Fremdkörpern schützenden, anatomisch angepasst bemessenen manschettenartigen zumindest teilelastischen Einrichtung versehen ist. Die Einrichtung kann ebenso wie vorstehend bezüglich der Schutzeinrichtung zum Schutz einer äußeren Prothese beschrieben weiter ausgebildet sein oder werden.

Gegenüber bekannten Badeprothesen weist die vorliegende Schutzeinrichtung somit den Vorteil auf, sehr viel handlicher und kleiner ausgebildet zu sein, gegenüber den ansonsten sperrigen Badeprothesen, was insbesondere auf Reisen ein großer Vorteil ist. Es muss nicht zusätzlich eine Badeprothese im Reisegepäck mit verstaut werden, sondern lediglich die klein zusammenfaltbare Schutzeinrichtung, die wie ein normales Bekleidungsstück im Reisegepäck Platz findet. Auch müssen weder ein Pumpball noch eine Wärme abstrahlende Einrichtung zum Schrumpfen und somit Anlegen der Schutzeinrichtungen an die Haut der diese tragenden Person mitgenommen werden, was ebenfalls einen großen Vorteil gegenüber den in Einheitsgrößen ausgebildeten schlauchförmigen Schutzeinrichtungen des Standes der Technik darstellt. Diese Vorteile ergeben sich nicht nur auf Reisen, sondern auch bei der sonstigen Freizeitgestaltung, insbesondere beim Freizeitsport, da auch hier die Schutzeinrichtung sehr viel einfacher im Sportgepäck mitgeführt werden kann als dies insbesondere bei Badeprothesen, jedoch auch bei den anderen evakuierbaren und schrumpfbaren schlauchförmigen Schutzeinrichtungen der Fall ist. Auch gegenüber den anderen vorstehend genannten Schutzeinrichtungen des Standes der Technik ist die erfindungsgemäße Schutzeinrichtung komfortabler bei der Verwendung, also auch beim An- und Ausziehen, bei der Pflege und Reinigung. Auch ggf. erforderlich werdende Reparaturen können einfacher durchgeführt werden, da einerseits das mehrlagige Material besser repariert werden kann und andererseits aufgrund des Anfügens der manschettenartigen Einrichtung sogar diese bei Verschleiß oder Beschädigung leicht ausgetauscht oder repariert werden kann.

Zur näheren Erläuterung der Erfindung werden im Folgenden Ausführungsbeispiele von dieser näher anhand der Zeichnungen beschrieben. Diese zeigen in:
- Figur 1a: eine Seitenansicht einer ersten Ausführungsform einer Schutzeinrichtung zur Verwendung für Armprothesen,
- Figur 1b: eine Seitenansicht einer Variante der Ausführungsform der Schutzeinrichtung nach Figur 1a,
- Figur 2a: eine Seitenansicht auf eine zweite Ausführungsform einer Schutzeinrichtung zur Verwendung für eine Armprothese,
- Figur 2b: eine Seitenansicht einer Variante der Ausführungsform der Schutzeinrichtung nach Figur 2a,
- Figur 3: eine Seitenansicht einer Ausführungsform einer Schutzeinrichtung zur Verwendung für eine Beinprothese,
- Figur 4: eine Seitenansicht einer weiteren Ausführungsform einer Schutzeinrichtung zur Verwendung für eine Beinprothese,
- Figur 5: eine Seitenansicht einer siebten Ausführungsform einer Schutzeinrichtung, zur Verwendung für eine Armprothese,
- Figur 6: eine Seitenansicht einer achten Ausführungsform einer Schutzeinrichtung, zur Verwendung für eine Beinprothese,
- Figur 7: eine Seitenansicht einer neunten Ausführungsform einer Schutzeinrichtung, zur Verwendung für eine Armprothese, in der Ausbildung als oberkörperintegrierender Schutzüberzug, und
- Figur 8: eine Seitenansicht einer zehnten Ausführungsform einer Schutzeinrichtung, zur Verwendung für eine Beinprothese, in Ausbildung als hosenartiger Schutzüberzug.

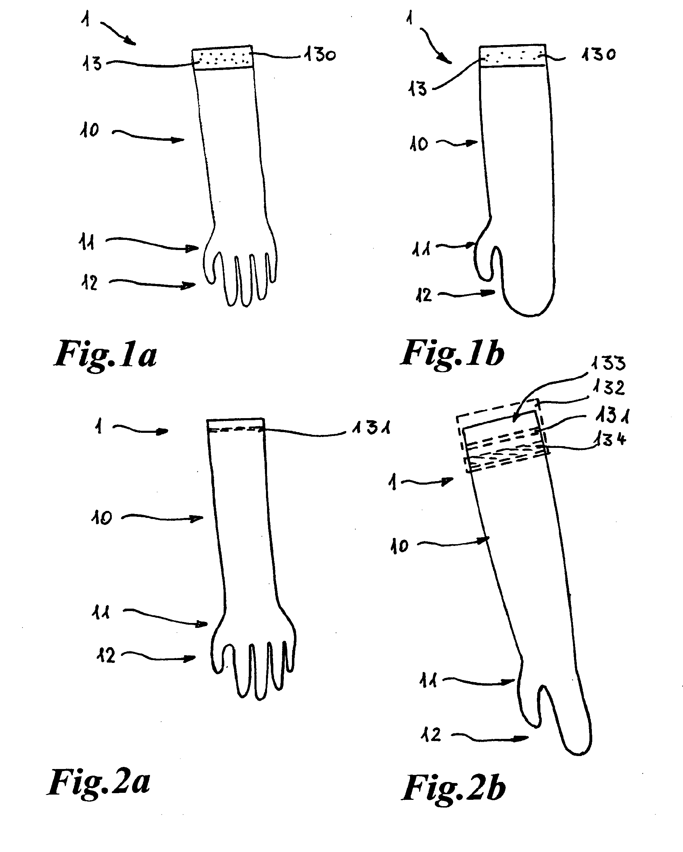

Figuren 1a und 1b zeigen Ausführungsformen einer Schutzeinrichtung 1 in Form eines sog. Armlings, also einen Schutzüberzug für eine Armprothese. Die Schutzeinrichtung weist sowohl einen Armabschnitt 10 als auch einen Handabschnitt 11 mit Fingerabschnitt 12, der in der Ausführungsform nach Figur 1 a als ausgeprägte Finger und in der Ausführungsform nach Figur 1b lediglich als angedeutete Finger bzw. als Fäustling ausgeführt ist, auf. Der Fingerabschnitt überdeckt wie ein Handschuh die Finger der Handprothese, ebenso wie der Handabschnitt der Schutzeinrichtung 1 den übrigen Teil der Handprothese überdeckt. Der Armabschnitt überdeckt sowohl den Armteil der Prothese als auch einen Teil des Extremitätenstumpfes der die Prothese tragenden Person, liegt also auf deren Haut auf. Die Auflage auf der Haut erfolgt zumindest in dem Bereich eines Oberarmabschlusses 13. Dieser ist auf zumindest der Innenseite mit einem haftenden Material versehen bzw. besteht aus einem solchen haftenden Material, um eine Wasserdichtigkeit und einen guten Halt hier vorsehen zu können. Beispielsweise weist die Schutzeinrichtung 1 im Bereich des Oberarmabschlusses 13 eine manschettenartige Einrichtung 130 in Form einer angefügten Manschette, auf. Diese kann aus Latex und/oder Silikon bestehen. Ferner kann eine andere glatte Hafteigenschaft aufweisende Oberfläche vorgesehen werden, die in Richtung des Extremitätenstumpfs angeordnet wird, also auf der Innenseite der Schutzeinrichtung 1 bzw. der manschettenartigen Einrichtung 130. Bei Vorsehen eines anderen Materials als Latex und/oder Silikon für den Oberarmabschluss 13 kann ggf. ein keilförmiger Einsatz bzw. ein Einsatz aus Latex und/oder Silikon vorgesehen werden, der den Halt auf dem Extremitätenstumpf bzw. der Haut einer die Schutzeinrichtung tragenden Person deutlich erhöht. Das Material der Schutzeinrichtung bzw. die manschettenartige Einrichtung 130 weist zumindest im Bereich des Armabschnitts in der Nähe des Oberarmschlusses und in dem Bereich des Oberarmschlusses eine Materialelastizität auf, um sich besonders gut und dicht an den Extremitätenstumpf anlegen zu können, also hier einen dichten Abschluss auf der Haut der die Prothese tragenden Person vorsehen zu können.

Figuren 2a und 2b zeigen eine zweite Ausführungsform einer Schutzeinrichtung 1 in Form eines Armlings, wobei diese Ausführungsform im Prinzip sehr ähnlich der in Figuren 1a und 1b gezeigten ist. Im Unterschied zu diesen weist die Schutzeinrichtung 1 gemäß Figur 2a und 2b jedoch keinen Oberarmabschluss mit angefügtem Latex und/oder Silikon auf. Vielmehr ist der Oberarmabschluss in Form der manschettenartigen Einrichtung 130 auf der zu der die Schutzeinrichtung tragenden Person gerichteten Innenseite mit einer glatten Hafteigenschaft aufweisenden Oberfläche versehen, insbesondere aus einem sog. Glatthaut-Neopren^{®} hergestellt. Der Oberarmabschluss kann als Manschette an das übrige Material der Schutzeinrichtung angesetzt oder ggf. mit diesem einteilig hergestellt sein. Zum Erzeugen einer Wasserdichtigkeit beim Aufliegen auf der Haut einer die Schutzeinrichtung 1 tragenden Person kann auf diese glatte bereits von sich aus Hafteigenschaft aufweisende Oberfläche zusätzlich ein Hautkleber 131 aufgefügt werden, der eine besonders sichere und dichte Verbindung mit der Haut der den Armling tragenden Person ermöglicht. Diese Variante findet insbesondere Anwendung bei Vorsehen von Glatthaut-Neopren^{®}, kann grundsätzlich auch bei einer angefügten Silikonmanschette vorgesehen werden. Ein Silikonmaterial erweist sich insbesondere bei Personen, die auf Latexmaterial reagieren, or allergisch auf dieses reagieren, als vorteilhaft. Allerdings kann durch Vorsehen einer Latex- oder Silikonmanschette bereits ein zusätzliches Maß an Wasserdichtigkeit geschaffen werden, so dass weitere Hilfsmittel hier zum Erzeugen eines Schutzes gegen das Eindringen von Wasser nicht unbedingt erforderlich sind, vorallem, wenn lediglich ein Spritzwasserschutz gewünscht wird.

Anstelle eines Hautklebers kann ein manschettenartiges Abdeckelement 132 über dem Randbereich der Schutzeinrichtung und die Haut der diese tragenden Person gezogen werden, um einen guten Schutz gegen das Eindringen von Feuchtigkeit, Nässe und Staub oder Schmutz zu bieten. Ein solches Abdeckelement ist in Figur 2b lediglich angedeutet und durch gestrichelte Linien dargestellt. Zum besonders wasserdichten Verbinden kann die Schutzeinrichtung im Bereich des Oberarmabschlusses auf der Außenseite 133 mit einem haftfähigen Material z.B. in Form einer Bedruckung 134 versehen sein. Dies ist in Figur 2b angedeutet.

Für die Verwendung für einen rechten und linken Arm können entweder die gleichen Schutzeinrichtungen 1 vorgesehen werden oder entsprechend angepasst unterschiedlich ausgebildete Schutzeinrichtungen. Insbesondere können auf dem Handteller, gegebenenfalls auch im Bereich der Fingerabschnitte, Beschichtungen vorgesehen werden, die eine Rutschfestigkeit unterstützen. Hierdurch kann die die Prothese tragende Person sich auf der Prothesenhand gegebenenfalls abstützen, ohne befürchten zu müssen, gerade auf nassem, also glitschigem Untergrund, wegzurutschen.

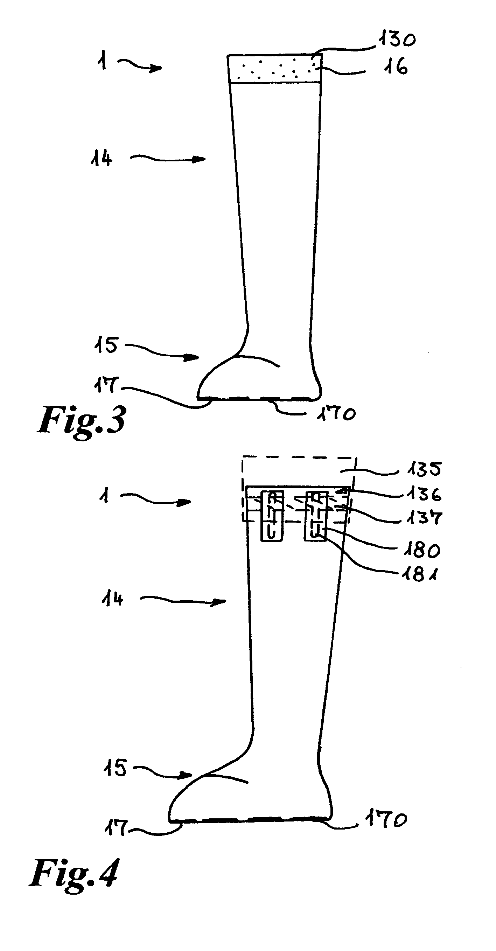

Figur 3 zeigt die Schutzeinrichtung 1 in Form eines sog. Beinlings zum Überziehen über eine Beinprothese. Diese Schutzeinrichtung ist entsprechend der Schutzeinrichtung 1 gemäß Figur 1 a und 1 b ausgebildet, jedoch mit einem Beinabschnitt 14 und einem Fußabschnitt 15 versehen. Anstelle eines Oberarmabschlusses 13 ist hier ein Oberschenkelabschluss 16 vorgesehen, der ebenfalls einen Schutz vor dem Eindringen von Wasser und Fremdkörpern ggf. bereits ohne das Vorsehen eines Hautklebers sorgt. Zu diesem Zweck weist der Oberschenkelabschluss im Bereich der manschettenartigen Einrichtung 130 wiederum eine Hafteigenschaft aufweisende Oberfläche auf. Diese besteht insbesondere aus einem lang anhaltend elastischen Material mit einer Haftwirkung. Ebenso wie der Oberarmabschluss 13 ist auch der Oberschenkelabschluss 16 zumeist als separates Element an dem Beinabschnitt 14 bzw. Armabschnitt 11 angefügt. Beispielsweise besteht der Oberschenkelabschluss 16 aus Latex und/oder Silikon oder weist einen entsprechenden Einsatz aus Latex und/oder Silikon auf. Ebenso ist es möglich, den Oberschenkelabschluss bzw. Oberarmabschluss im Bereich der manschettenartigen Einrichtung 130 auf der zur Haut des Trägers gerichteten Seite haftend zu beschichten bzw. zu kaschieren oder ein entsprechende Hafteigenschaften aufweisendes Material zu verwenden, insbesondere Glatthaut-Neopren^{®}.

Eine solche zu der in Figur 3 gezeigten Ausführungsform eines Beinlings alternative Ausführungsform ist in Figur 4 gezeigt. Der hier dargestellte Beinling unterscheidet sich von der Ausführungsform nach Figur 3 lediglich darin, dass eine innere, also zu der die Schutzeinrichtung tragenden Person gerichtete glatte, Hafteigenschaft aufweisende Oberfläche in dem Oberschenkelabschlussbereich bzw. dem Bereich der manschettenartigen Einrichtung vorgesehen ist, insbesondere ein sog. Glatthaut-Neopren^{®}. Der Oberschenkelabschluss kann als Manschette an das übrige Material der Schutzeinrichtung angesetzt oder ggf. mit diesem einteilig hergestellt sein. Wie bei der Ausführungsform gemäß Figur 2a und 2b kann auch hier ein Hautkleber genutzt werden, um eine noch bessere Wasserdichtigkeit beim Auffügen auf die Haut des Prothesenträgers vorzusehen. Ggf. kann auch bereits das Vorsehen eines Keileinsatzes aus Latex und/oder Silikon für das Schaffen einer Wasserdichtigkeit ausreichen. Eine weitere alternative Ausführungsvariante besteht darin, ein separates manschettenartiges Abdeckelement 135 z.B. aus Latex und/oder Silikon vorzusehen. Dieses wird über den Randbereich der Schutzeinrichtung und über die Haut der Person, die die Schutzeinrichtung trägt, gezogen und kann hierdurch ein Eindringen von insbesondere Feuchtigkeit, Nässe sowie Staub und Schmutz in die Schutzeinrichtung verhindern, wie bereits vorstehend zu den Figuren 2a und 2b erwähnt. Ebenso wie dort ist auch hier eine zusätzliche Abdichtung durch z.B. Bedrucken der Außenseite 136 der Schutzeinrichtung mit einem haftfähigen Material in Form einer Bedruckung 137 im Bereich des Oberschenkelabschlusses möglich (in Figur 4 lediglich angedeutet).

Um ein Aufrollen des Oberschenkelabschlusses zu vermeiden, sind dort mehrere taschenartige Aufrollverhinderungseinrichtungen 180 mit stabartigen Stützeinlagen 181 aufgefügt. Die Stützeinlagen 181 können zum Reinigen entnommen werden, weswegen die taschenartigen Aufrollverhinderungseinrichtungen 180 einseitig offen sind. Sie können jedoch auch vollständig geschlossen sein, so dass eine Entnahme der Stützeinlagen dann nicht mehr möglich ist. Die Stützeinlagen 181 können auch direkt auf das Material der Schutzeinrichtung im Bereich des Oberschenkelabschlusses aufgefügt, z.B. dort auf- oder eingenäht werden, wobei dann eine Entnahme ebenfalls nicht mehr möglich ist.

Bei den Ausführungsformen der Schutzeinrichtung nach Figur 3 und 4 können jeweils die Fußsohlen 17 der beiden Beinlinge mit einer Rutschfestigkeit vorsehenden Beschichtung 170 versehen sein. Gerade auf nassem bzw. rutschigem Untergrund kann hierdurch ein besserer Halt für die die Prothese tragende Person geschaffen werden.

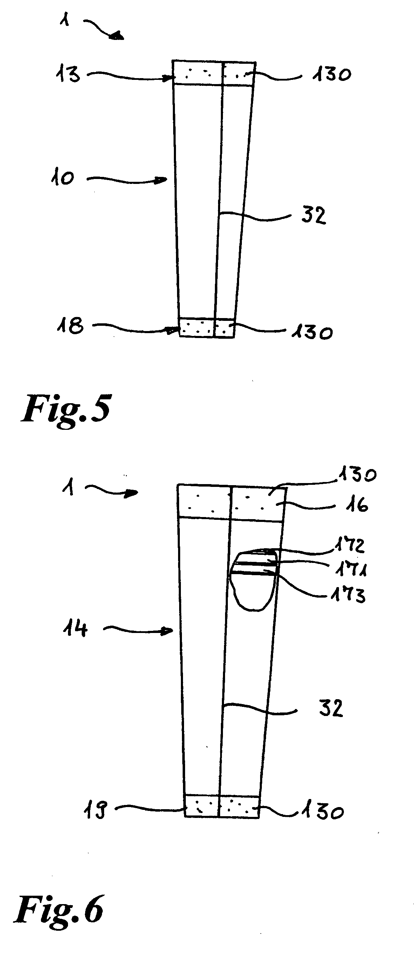

Nachdem in den Figuren 1a, 1b und 2a, 2b jeweils Armlinge mit angefügtem beziehungsweise integriertem Handabschnitt 11 und Fingerabschnitt 12 gezeigt sind, weist die Schutzeinrichtung 1 gemäß Figur 5 weder einen Hand- noch einen Fingerabschnitt auf. Diese Schutzeinrichtung ist lediglich schlauchförmig ausgebildet als Armabschnitt 10. Auch diese Ausführungsform weist jedoch, genauso wie die Ausführungsform nach Figur 1, einen Oberarmabschluss 13 mit einer manschettenartigen Einrichtung 130 auf. Zusätzlich ist zum Abdichten im Bereich eines Handgelenks der Armprothese noch ein Handgelenksabschluss 18 vorgesehen. Der Oberarmabschluss 13 ist wiederum vorteilhaft aus einem lang anhaltend elastischen Material mit einer Haftwirkung, insbesondere Latex und/oder Silikon ausgebildet bzw. mit einem Haftwirkung zeigenden Material und ggf. einem Hautkleber versehen, um einen gegen das Eindringen von Wasser abdichtenden Abschluss auf der Haut der die Prothese tragenden Person zu schaffen. Da der Handgelenkabschluss 18 auf der Prothesenoberfläche aufliegt, die Prothesenhand also unbedeckt bleibt, wird im Bereich des Handgelenkabschlusses 18 ein dichtender Abschluss gegenüber der Oberfläche der Prothese geschaffen, wobei auch hier wiederum vorteilhaft ein langanhaltend elastisches Material mit einer Haftwirkung verwendet wird.

Eine entsprechende Beinlingsausführung ohne Fußabschnitt ist in Figur 6 zu sehen. Hier bleibt der Prothesenfuß der diese Prothese tragenden Person unbedeckt, wobei der Beinling im Bereich des Sprunggelenks der Prothese einen Sprunggelenksabschluss 19 aufweist. Somit umfasst die Schutzeinrichtung 1 gemäß Figur 6 lediglich einen Beinabschnitt 14 mit endseitigem Oberschenkelabschluss 16 und Sprunggelenkabschluss 19, somit zwei Öffnungen, die wasserdicht einerseits an der Haut der die Prothese tragenden Person, andererseits an der Prothese selbst anliegen. Auch hier können im Bereich des Oberschenkelabschlusses sowie des Sprunggelenkabschlusses wiederum langanhaltend elastische Materialien mit einer Haftwirkung, insbesondere als Vollmaterial, z.B. Latex und/oder Silikon, vorgesehen werden in Form von manschettenartigen Einrichtungen 130.

Sowohl der Armling gemäß Figur 5 als auch der Beinling gemäß Figur 6 können an ihren beiden einander gegenüberliegenden Enden auch lediglich mit einer glatten, zumindest ein geringes Maß an Hafteigenschaft aufweisenden Oberfläche versehen sein, und können wasserdicht durch Verwendung eines Hautklebers auf die Haut bzw. ggf. auf die Prothesenoberfläche aufgeklebt zu werden. Allerdings eignet sich gerade im Bereich der Prothese eher das Vorsehen eines haftenden Materials, das ohne die Verwendung eines Klebemittels auskommt, da letzteres bei Entfernen der Schutzeinrichtung wieder von der Prothese entfernt werden müsste, ohne diese in irgendeiner Form zu beschädigen. Daher eignet sich eher die Verwendung von selbständig haftenden Materialien, die rückstandsfrei von der Oberfläche der Prothese wieder entfernt werden können, ohne diese anzugreifen, wie z.B. von Latex bzw. Silikon. Geeignet ist hier daher insbesondere ein manschettenartiges Abdeckelement aus z.B. Latex und/oder Silikon, das die Schutzeinrichtung im jeweiligen Randbereich und die nicht überdeckte Prothese überdeckend angeordnet wird.

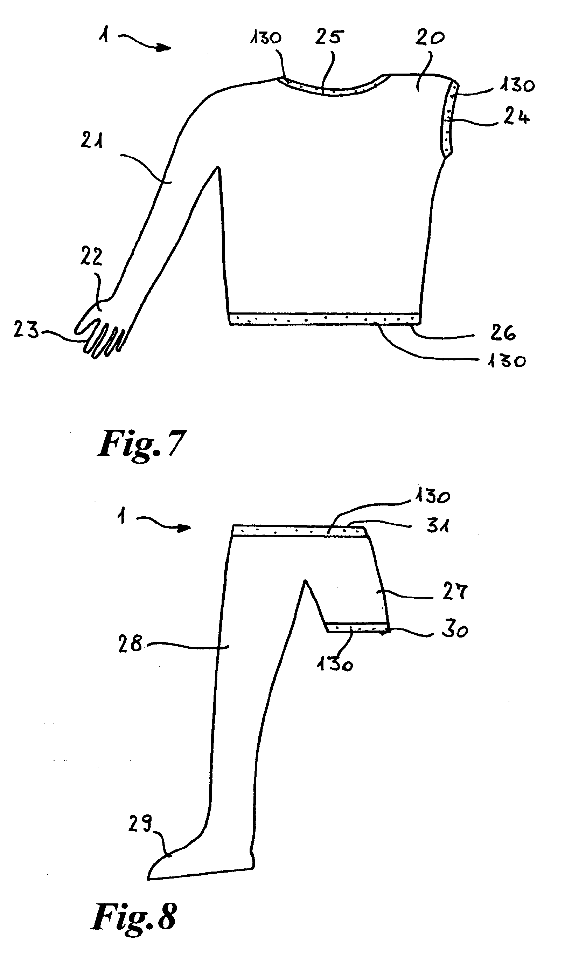

Figur 7 zeigt eine weitere Ausführungsform einer Schutzeinrichtung 1, die hier als oberkörperintegrierender Schutzüberzug ausgebildet ist. Dieser ist ähnlich wie ein Pullover geformt, bei dem einseitig ein Ärmel mit Handabschnitt angeformt ist, ein zweiter Ärmel jedoch weggelassen ist. Somit weist die Schutzeinrichtung 1 einen Oberkörperabschnitt 20, einen Armabschnitt 21 sowie einen Handabschnitt 22 mit Fingerabschnitt 23 auf. Die auf der Seite des Armabschnitts 21, Handabschnitts 22 und Fingerabschnitts 23 angeordnete Armprothese kann somit vollständig aufgenommen und geschützt werden. Gerade dann, wenn der Extremitätenstumpf zu kurz ausgebildet ist, um hieran sicher eine Schutzeinrichtung zu befestigen, erfolgt eine Befestigung in der Form, dass durch den Oberkörperabschnitt 20 ein sehr guter Halt am Körper der die Prothese tragenden Person geschaffen werden kann. Der gesunde Arm durchragt eine entsprechende Armöffnung 24 in dem Oberkörperabschnitt. Ferner weist der Oberkörperabschnitt 20 eine Halsöffnung 25 sowie eine untere zum Unterkörper gerichtete Öffnung 26 auf. Im Bereich dieser drei Öffnungen 24 bis 26 sind vorteilhaft manschettenartige Einrichtungen 130 zum Schutz vor dem Eintritt von Nässe, Feuchtigkeit sowie Schmutz vorgesehen, insbesondere ein langanhaltend elastisches Material mit einer zumindest einseitigen Haftwirkung, wie Glatthaut-Neopren^{®} oder als Vollmaterial Latex oder Silikon, wobei Hafteigenschaft aufweisende Materialbeschichtungen oder Kaschierungen auf ein anderes Material aufgefügt werden können. Ebenfalls ist es möglich, beispielsweise einen Chloropren-Kautschuk, Polychloropren- oder Chlorbutadien-Kautschuk mit einer glatten, Hafteigenschaft aufweisenden Oberfläche zu verwenden, wobei auch hier zusätzlich ein Hautkleber zum wasserdichten Abschluss im Bereich der Öffnungen 24 bis 26 aufgetragen werden kann.

Ein entsprechendes Pendant zu der Ausführungsform für eine Armprothese ist in Figur 8 für eine Beinprothese in Form der einseitig hosenartig ausgebildeten Schutzeinrichtung 1 gezeigt. Ein Bein dieser hosenartigen Schutzeinrichtung ist dabei nach Art einer Strumpfhose ausgebildet, das andere erstreckt sich hingegen lediglich mit einem Oberschenkelabschnitt 27 über einen Teil des gesunden Oberschenkels einer einseitig eine Beinprothese tragenden Person. Die Beinprothese wird in dem strumpfhosenartig ausgebildeten Beinabschnitt 28 vollständig aufgenommen. Der Beinabschnitt weist zudem einen Fußabschnitt 29 auf. Somit ist hier ein vollständiges Umhüllen der Beinprothese auch in dem Falle möglich, dass der Extremitätenstumpf im Oberschenkelbereich zu kurz ist, um daran eine Schutzeinrichtung nach Figur 3 oder 4 befestigen zu können, so dass ein sicherer Schutz für die Beinprothese ermöglicht werden kann.

Die beiden verbleibenden Öffnungen 30, 31 am Oberschenkelabschnitt und im Bereich der Hüfte der die Schutzeinrichtung tragenden Person können wiederum in verschiedener Art und Weise zum Erzeugen einer Wasserdichtigkeit ausgebildet sein, insbesondere manschettenartige Einrichtungen 130 aus einem langanhaltend elastischen Material mit einer Haftwirkung vorgesehen sein bzw. eine glatte, Hafteigenschaft aufweisende Oberfläche aufweisen und gegebenenfalls mit einem Hautkleber auf der Hautoberfläche der die Schutzeinrichtung tragenden Person verklebt sein. Ferner ist das Vorsehen z.B. einer Manschette aus Latex und/oder Silikon möglich.

Gerade bei den Ausführungsformen gemäß Figur 7 und 8 erweist sich eine an die Körperform der die Schutzeinrichtung tragenden Person angepasste Formgebung als besonders vorteilhaft, da einerseits ein dichtes Anliegen des Materials auf der Hautoberfläche der Person dazu führt, dass die gewünschte Dichtheit sichergestellt werden kann und andererseits diese Person nicht unnötig durch die Schutzeinrichtung behindert wird.

Neben den gezeigten Formgebungen können noch zahlreiche weitere gebildet werden, insbesondere auch Mischformen der gezeigten Ausführungsvarianten. Insbesondere können auch die beiden Ausführungsformen gemäß Figur 7 und 8 jeweils ohne Handabschnitt bzw. ohne Fußabschnitt ausgebildet werden, also entsprechend einen Handgelenkabschluss bzw. Sprunggelenkabschluss aufweisen. In jedem Fall erweist es sich als vorteilhaft, einen sicheren Halt am Körper der die Prothese tragenden Person vorzusehen, um die hochwertige Prothese gegen Beschädigung insbesondere durch eindringenden Staub, Sand bzw. Wasser, insbesondere Meerwasser, zu schützen. Auch ein verletzter Arm oder ein verletztes Bein können durch eine solche Schutzeinrichtung vor dem Kontakt mit Feuchtigkeit und Fremdkörpern geschützt werden.

Als Material für die Schutzeinrichtung eignet sich ein Chloropren-Kautschuk, Polychloropren- oder Chlorbutadien-Kautschuk, insbesondere in geschäumter Form, wobei eine Mehrlagigkeit des Materials der Schutzeinrichtung vorgesehen ist, wie in Figur 6 angedeutet. Die Lage 171 aus geschäumtem Material kann in diesem Fall besonders gut als innere Materialschicht vorgesehen werden, oberhalb und unterhalb derer weitere Materialschichten 172, 173 angeordnet werden, die eine höhere Verschleißfestigkeit bieten. Derartige Schichten können beispielsweise aus Lycra und/oder Nylon oder anderen verschleißfesten Materialien bestehen. Der Materialzuschnitt wird zum Schaffen einer dichten Schutzeinrichtung vernäht, verklebt, verschweißt oder in anderer Weise dichtend verschlossen. Eine derartige dichte Naht 32 ist in Figur 5 und 6 angedeutet. Die Oberfläche der Schutzeinrichtung kann eingefärbt werden, insbesondere individuell auf die Hautfarbe der die Prothese tragenden Person. Auch eine Kaschierung oder Beschichtung selbst kann eingefärbt werden. Beim Einfärben können sogar Körperbehaarungen nachempfunden werden.

Neben den vorstehend beschriebenen und in den Figuren dargestellten Ausführungsformen von Schutzeinrichtungen zur Verwendung für äußere Prothesen können noch zahlreiche weitere gebildet werden, bei denen jeweils die Schutzeinrichtung ein maßlich anatomisch angepasster Schutzüberzug aus einem zumindest teilelastischen, und wasserundurchlässigen oder im Wesentlichen wasserundurchlässigen oder vor Wassereintritt oder Spritzwasser schützenden bzw. wasserfesten, widerstandsfähigen, reißfesten bzw. beschädigungsresistenten Material ist und zumindest an einem Ende mit einer vor Eintritt von Nässe oder Feuchtigkeit sowie Fremdkörpern schützenden manschettenartigen zumindest teilelastischen Einrichtung versehen ist, die anatomisch angepasst an die die Schutzeinrichtung tragende Person bemessen ist und insbesondere haftend auf der Haut der Person Halt finden und ein Eindringen von Wasser oder Feuchtigkeit bzw. Schmutz oder Staub sowie anderen Fremdkörpern in das Innere der Schutzeinrichtung verhindern kann.

### Bezugszeichenliste

- 1: Schutzeinrichtung
- 10: Armabschnitt
- 11: Handabschnitt
- 12: Fingerabschnitt
- 13: Oberarmabschluss
- 14: Beinabschnitt
- 15: Fußabschnitt
- 16: Oberschenkelabschluss
- 17: Fußsohle
- 18: Handgelenkabschluss
- 19: Sprunggelenkabschluss
- 20: Oberkörperabschnitt
- 21: Armabschnitt
- 22: Handabschnitt
- 23: Fingerabschnitt
- 24: Armöffnung
- 25: Halsöffnung
- 26: untere Öffnung
- 27: Oberschenkelabschnitt
- 28: Beinabschnitt
- 29: Fußabschnitt
- 30: Öffnung
- 31: Öffnung
- 32: Naht
- 130: manschettenartige Einrichtung
- 131: Hautkleber
- 132: Abdeckelement
- 133: Außenseite
- 134: Bedruckung mit haftfähigem Material
- 135: Abdeckelement
- 136: Außenseite
- 137: Bedruckung mit haftfähigem Material
- 170: Rutschfestigkeitsbeschichtung
- 171: Lage aus geschäumtem Material/innere Materialschicht
- 172: obere Materialschicht
- 173: untere Materialschicht
- 180: taschenartige Aufrollverhinderungseinrichtung
- 181: stabartige Stützeinlage

## Patentansprüche

1. Schutzeinrichtung (1) zur Verwendung für äußere Prothesen, wobei die Schutzeinrichtung ein maßlich anatomisch angepasster Schutzüberzug ist,
der Schutzüberzug zumindest als Hand oder Fuß integrierender Arm- oder Beinling ausgebildet ist, aus einem zumindest teilelastischen, wasserundurchlässigen oder anwendungsspezifisch ausreichend ein Eindringen von Wasser in das Innere des Schutzüberzugs verhindernden, widerstandsfähigen und beschädigungsresistenten Material besteht, und zumindest an einem Ende mit einer vor Eintritt von Nässe, Feuchtigkeit oder Fremdkörpern schützenden, anatomisch angepasst bemessenen manschettenartigen, zumindest teilelastischen, eine glatte, Hafteigenschaft aufweisende Oberfläche aufweisenden Einrichtung (130) versehen ist, **dadurch gekennzeichnet, dass** das Material der Schutzeinrichtung mehrlagig ausgebildet ist.

2. Schutzeinrichtung (1) nach Anspruch 1,
**dadurch gekennzeichnet, dass**
die manschettenartige Einrichtung (130) ein langanhaltend elastisches Material mit einer Adhäsivwirkung, insbesondere Latex und/oder Silikon, und/oder ein Material mit glatter, Hafteigenschaft aufweisender Oberfläche umfasst, insbesondere Chloropren-Kautschuk, Polychloropren- oder Chlorobutadien-Kautschuk.

3. Schutzeinrichtung (1) nach einem der vorstehenden Ansprüche ,
**dadurch gekennzeichnet, dass**
die manschettenartige Einrichtung (130) an das Material der Schutzeinrichtung (1) angesetzt ist.

4. Schutzeinrichtung (1) nach Anspruch 1, 2 oder 3,
**dadurch gekennzeichnet, dass**
ein Hautkleber (131) zum Erreichen einer Wasserdichtigkeit vorgesehen ist.

5. Schutzeinrichtung (1) nach einem der vorstehenden Ansprüche,
**dadurch gekennzeichnet, dass**
zumindest eine geschäumte Lage (171) vorgesehen ist.

6. Schutzeinrichtung (1) nach einem der vorstehenden Ansprüche,
**dadurch gekennzeichnet, dass**
die Schutzeinrichtung aus einem wasserdichten, nicht wasseraufnehmenden Material besteht, insbesondere aus einem Chloropren-Kautschuk, Polychloropren- oder Chlorbutadien-Kautschuk.

7. Schutzeinrichtung (1) nach einem der vorstehenden Ansprüche,
**dadurch gekennzeichnet, dass**
ein separates Abdeckelement (132, 135) aus einem wasserdichten oder ein Eindringen von Wasser verhindernden Material vorgesehen ist.

8. Schutzeinrichtung (1) nach einem der vorstehenden Ansprüche,
**dadurch gekennzeichnet, dass**
die manschettenartige Einrichtung (130) zumindest eine eine Aufrollbewegung verhindernde Einrichtung (180,181) aufweist, insbesondere zumindest eine taschenartige Aufrollverhinderungseinrichtung (180) mit zumindest einer stabartigen Stützeinlage (181).

9. Schutzeinrichtung (1) nach einem der vorstehenden Ansprüche,
**dadurch gekennzeichnet, dass**
die Schutzeinrichtung zumindest einseitig oberflächenbeschichtet, insbesondere kaschiert ist.

10. Schutzeinrichtung (1) nach einem der vorstehenden Ansprüche,
**dadurch gekennzeichnet, dass**
Stoßstellen (32) des Materials im Wesentlichen wasserdicht verschlossen sind, insbesondere vernäht, verschweißt oder verklebt sind.

11. Schutzeinrichtung (1) nach einem der vorstehenden Ansprüche,
**dadurch gekennzeichnet, dass**
im Bereich der Sohle eines Fußes der Schutzeinrichtung (1) zumindest ein Reibung erhöhendes Material (170) oder Materialflecken vorgesehen ist.

12. Schutzeinrichtung (1) nach einem der Ansprüche 1 bis 11,
**dadurch gekennzeichnet, dass**
die Schutzeinrichtung (1) als Teil des Körpers der die Schutzeinrichtung tragenden Person integrierender einbeiniger hosenartiger oder oberkörperintegrierender Schutzüberzug ausgebildet ist.

## Claims

1. Protective arrangement (1) for use for external prostheses, wherein said protective arrangement is a protective covering which is anatomically adapted in terms of dimensions, said protective covering is constructed at least as a small arm or leg incorporating a hand or foot and consists of a material which is at least partly elastic, is water-impermeable or adequately prevents the penetration of water into the interior of the protective covering in a manner specific to the application, and is robust and resistant to damage, and said protective covering is provided, at least at one end, with a sleeve-like and at least partly elastic arrangement (130) which provides protection against the ingress of wetness, dampness or foreign bodies, is dimensioned in an anatomically adapted manner and has a smooth surface having an adhering property, **characterised in that** the material of the protective arrangement is of multilayer construction.

2. Protective arrangement (1) according to Claim 1,
**characterised in that**
the sleeve-like arrangement (130) comprises an enduringly elastic material with an adhesive action, in particular latex and/or silicone, and/or a material with a smooth surface having an adhering property, in particular chloroprene rubber, polychloroprene rubber or chlorobutadiene rubber.

3. Protective arrangement (1) according to one of the preceding claims,
**characterised in that**
the sleeve-like arrangement (130) is attached to the material of the protective arrangement (1).

4. Protective arrangement (1) according to Claim 1, 2 or 3,
**characterised in that**
a skin adhesive (131) is provided for achieving water-tightness.

5. Protective arrangement (1) according to one of the preceding claims,
**characterised in that**
at least one foamed layer (171) is provided.

6. Protective arrangement (1) according to one of the preceding claims,
**characterised in that**
the protective arrangement consists of a watertight material which does not absorb water, in particular of a chloroprene rubber, polychloroprene rubber or chlorobutadiene rubber.

7. Protective arrangement (1) according to one of the preceding claims,
**characterised in that**
a separate covering element (132, 135) made of a material which is watertight or which prevents the penetration of water is provided.

8. Protective arrangement (1) according to one of the preceding claims,
**characterised in that**
the sleeve-like arrangement (130) has at least one arrangement (180, 181) which prevents a rolling-up movement, in particular at least one pocket-like roll-up-preventing arrangement (180) with at least one bar-like supporting insert (181).

9. Protective arrangement (1) according to one of the preceding claims,
**characterised in that**
the protective arrangement is surface-coated, in particular laminated, at least on one side.

10. Protective arrangement (1) according to one of the preceding claims,
**characterised in that**
points of impact (32) in the material are sealed, in particular sewn up, welded or bonded, in a substantially watertight manner.

11. Protective arrangement (1) according to one of the preceding claims,
**characterised in that**
at least one friction-increasing material (170) or patches of material is/are provided in the region of the sole of a foot belonging to the protective arrangement (1).

12. Protective arrangement (1) according to one of Claims 1 to 11,
**characterised in that**
the protective arrangement (1) is constructed as a single-legged, trouser-like or thorax-incorporating protective covering which incorporates part of the body of the person wearing the protective arrangement.

## Revendications

1. Dispositif de protection (1) destiné à être utilisé pour des prothèses externes, le dispositif de protection étant un revêtement de protection adapté anatomiquement au niveau des dimensions, le revêtement de protection étant réalisé au moins comme une manchette ou une jambière intégrant une main ou un pied, constitué d'un matériau robuste, résistant aux endommagements, empêchant suffisamment de manière spécifique à l'utilisation une pénétration d'eau à l'intérieur du revêtement de protection ou imperméable à l'eau, au moins partiellement élastique, et étant pourvu au moins sur une extrémité d'un dispositif (130) présentant une surface lisse, présentant une propriété adhésive, au moins partiellement élastique, de type manchette aux dimensions adaptées anatomiquement, protégeant d'une pénétration humide, d'humidité ou de corps étrangers, **caractérisé en ce que** le matériau du dispositif de protection est réalisé à plusieurs couches.

2. Dispositif de protection (1) selon la revendication 1,
**caractérisé en ce**
**que** le dispositif (130) de type manchette comporte un matériau durablement élastique avec une action adhésive, en particulier du latex et/ou de la silicone, et/ou comporte un matériau avec une surface lisse, présentant une propriété adhésive, en particulier un caoutchouc chloroprène, un caoutchouc polychloroprène ou chlorobutadiène.

3. Dispositif de protection (1) selon l'une quelconque des revendications précédentes,
**caractérisé en ce**
**que** le dispositif (130) de type manchette est placé sur le matériau du dispositif de protection (1).

4. Dispositif de protection (1) selon la revendication 1, 2 ou 3,
**caractérisé en ce**
**qu'**une colle cutanée (131) est prévue pour obtenir une étanchéité à l'eau.

5. Dispositif de protection (1) selon l'une quelconque des revendications précédentes,
**caractérisé en ce**
**qu'**au moins une couche expansée (171) est prévue.

6. Dispositif de protection (1) selon l'une quelconque des revendications précédentes, **caractérisé en ce que** le dispositif de protection se compose d'un matériau étanche à l'eau, n'absorbant pas l'eau, en particulier d'un caoutchouc chloroprène, un caoutchouc polychloroprène ou chlorobutadiène.

7. Dispositif de protection (1) selon l'une quelconque des revendications précédentes,
**caractérisé en ce**
**qu'**un élément de recouvrement (132, 135) séparé en un matériau étanche à l'eau ou empêchant une pénétration d'eau est prévu.

8. Dispositif de protection (1) selon l'une quelconque des revendications précédentes,
**caractérisé en ce**
**que** le dispositif (130) de type manchette présente au moins un dispositif (180, 181) empêchant un mouvement d'enroulement, en particulier au moins un dispositif d'empêchement d'enroulement (180) de type poche avec au moins une couche intermédiaire d'appui (181) de type baguette.

9. Dispositif de protection (1) selon l'une quelconque des revendications précédentes,
**caractérisé en ce**
**que** la surface du dispositif de protection est revêtue au moins d'un côté, en particulier est stratifiée.

10. Dispositif de protection (1) selon l'une quelconque des revendications précédentes,
**caractérisé en ce**
**que** des points d'aboutement (32) du matériau sont fermés de manière sensiblement étanche à l'eau, en particulier sont cousus, soudés ou collés.

11. Dispositif de protection (1) selon l'une quelconque des revendications précédentes,
**caractérisé en ce**
**qu'**au moins un matériau augmentant le frottement (170) ou des morceaux de matériau sont prévus dans la zone de la semelle d'un pied du dispositif de protection (1).

12. Dispositif de protection (1) selon l'une quelconque des revendications 1 à 11,
**caractérisé en ce**
**que** le dispositif de protection (1) est réalisé comme un revêtement de protection intégrant le buste ou de type pantalon à une jambe intégrant une partie du corps de la personne portant le dispositif de protection.
